# EUROPÄISCHE PATENTANMELDUNG

(11) **EP 1 797 969 A1**
(43) Veröffentlichungstag der Anmeldung: **20.06.2007**
(21) Anmeldenummer: 05027687.2
(22) Anmeldetag: 16.12.2005
(51) Int. Cl.: B08B 9/027, B08B 9/00, G01N 1/22

(54) **Verfahren und Vorrichtung zum Reinigen von Komponenten einer Kraftwerksanlage durch Einblasen eines Mediums sowie Messeinrichtung zur Messung des Reinheitsgrads des Mediums**

(71) Anmelder: SIEMENS AKTIENGESELLSCHAFT, 80333 München (DE)
(72) Erfinder: Elsen, Jan, 91334 Hemhofen (DE); Lönne, Rolf, 91352 Hallerndorf (DE)

(57) **Zusammenfassung**

Die Erfindung betrifft ein Verfahren zum Reinigen von Anlagenteilen (2.1.3, 2.2.3, 2.3, 2.4.2, 4.1a, 4.2, 4.3a, 4.3b, 4.4, 4.5a bis 4.5c) einer Kraftwerksanlage (1), wobei ein Medium kontinuierlich in einem geschlossenen Strömungskreis (K) durch ein oder mehrere zu reinigende Anlagenteile (2.1.3, 2.2.3, 2.3, 2.4.2, 4.1a, 4.2, 4.3a, 4.3b, 4.4, 4.5a bis 4.5c) geführt wird und eine Prüfung des Mediums auf seinen Reinheitsgrad in mindestens einem betrieblichen Anlagenteil (4.1a, 4.2, 4.3a, 4.3b, 4.4, 4.5a bis 4.5c) durchgeführt wird.

## Beschreibung

Die Erfindung bezieht sich auf ein Verfahren zum Reinigen von Komponenten einer Kraftwerksanlage, insbesondere einer Dampfturbinenanlage oder einer Gas- und Dampfturbinenanlage. Sie betrifft weiterhin eine Vorrichtung zum Reinigen von Komponenten einer Kraftwerksanlage, in welche ein Medium, insbesondere ein dampfförmiges Medium, eingeführt, insbesondere eingeblasen wird. Des Weiteren betrifft sie eine Messeinrichtung zur Messung des Reinheitsgrads des Mediums und zur Verwendung in der Vorrichtung zum Reinigen der Komponenten.

Für den Betrieb der Kraftwerksanlage sind an die Reinheit des Dampfes hohe Anforderungen gestellt. Insbesondere gilt es zu vermeiden, dass im Dampf partikelförmige Feststoffe mitgeführt werden. Solche Festkörperpartikel können zur Beschädigung der Anlagenteile, wie z. B. der Turbine, führen. Die Beschädigungen fallen dabei insbesondere an der Beschaufelung und den Labyrinthdichtungen der Turbine an.

Zum Schutz der Turbine und anderer Anlagenteile vor größeren Festkörperpartikeln werden Dampfsiebe vor Abfangschnellschlussventilen bzw. zwischen Abfangschnellschlussventilen und den Regelventilen in den Dampfleitungen installiert. Festkörperpartikel mit kleinerem Durchmesser hingegen passieren die Siebe. Sie können somit in die Turbine gelangen. Durch sie wird eine so genannte Festkörper-Erosion der Beschaufelung hervorgerufen. Dies führt zu Materialabtrag und Schaufelaufrauhung und dadurch zur Verschlechterung des Turbinenwirkungsgrades. Soweit die Festkörperpartikel durch die Turbine abgebremst werden und in der Turbine verbleiben, besteht die Gefahr, dass sie zu Aufrauhungen an der Beschaufelung führen und gegebenenfalls Ablagerungen in den Labyrinthdichtungen bilden. Durch diese Ablagerungen können Dichtelemente oder andere Komponenten beschädigt oder zerstört werden.

In Kesseln, Dampfleitungen oder sonstigen dampfführenden Komponenten, die in Dampfströmungsrichtung vor der Turbine liegen, entstehen Festkörperpartikel, insbesondere während der Neumontage von Kraftwerken. Auch im Zuge von Anlagenrevisionen oder Komponentenaustausch entstehen Festkörperpartikel. Bei diesen Festkörperpartikeln handelt es sich insbesondere um Walzhaut, Zunder, Eisenoxide sowie durch Wärmebehandlung der Anlagenkomponenten verursachte Korrosionsprodukte und Oxidationsschichten. Selbst bei größter Sorgfalt während der Montagearbeit ist es nicht mit Sicherheit zu vermeiden, dass Staub, Sand, Montagegegenstände und Montageabfälle in dampfführenden Komponenten oder Anlagenteilen der Anlage zurückbleiben. Die Verunreinigungen liegen zum Teil lose vor. Zum Teil haften sie an den Innenwänden der Anlagenteile an.

Im Allgemeinen müssen daher als dampfführende Komponenten der Kesselbereich, Dampfleitungen zwischen Kessel und Dampfturbine bzw. zwischen Kessel und Kondensator sowie Ventile vor jedem ersten Dampfanstoß einer Dampfturbine ausgeblasen oder ausgespült werden und somit von Partikeln, insbesondere von Festkörperpartikeln, z. B. Walzhaut, Zunder, Eisenoxiden, befreit werden.

Zum Reinigen der Kraftwerksanlage wird ein Spülverfahren eingesetzt. Nach erfolgtem Spülen oder Beizen der Anlage werden die dampfführenden Systeme mit Dampf gereinigt. Dieser wird durch Verdampfung von höchstreinem Wasser (Deionat) im Kessel erzeugt. Dabei wird der Kraftwerksanlage das höchstreine Wasser zugeführt, durch die entsprechenden Komponenten geführt und wieder entnommen. So werden beispielsweise vor jedem ersten Dampfanstoß einer Dampfturbine die dampfführenden Kesselbereiche und Dampfleitungen von Partikeln befreit, die zu einer Beschädigung und/oder einer Wirkungsgradbeeinträchtigung führen können. Dieser Reinigungsprozess unterliegt verschiedenen Parametern, wie z. B. Deionaterzeugungsleistung, -speicherkapazität und schnelle Deionatnachspeisung, Schallschutzmaßnahmen, resultierenden Betriebseinschränkungen, Nachweis der Partikelfreiheit des Dampfes, Rückbau von Provisorien, nachgeschalteten abhängigen Inbetriebsetzungsschritten.

Beim Reinigen der Anlagenteile sind an die Kraftwerksanlage anschließbare temporäre Reinigungsprovisorien vorgesehen, die der Kraftwerksanlage das Spülmedium zuführen und zumindest einen Teil des Spülmediums nach Durchströmen durch die Komponenten wieder entnehmen.

Bei herkömmlichen Verfahren erfolgt die Reinigung der dampfführenden Anlagenteile durch eine chemische Reinigung (wie z. B. Beizen) oder durch Ausblasen mit Dampf oder durch eine Kombination der Methoden.

Beim Beizen sind die Reinigungswässer mit einem Beizmittel, z. B. inhibierter Säure, Komplex-Bildner, z. B. Äthylendiamintetraessigsäure, belastet und können daher nicht in die Umwelt abgeführt werden. Für die Entsorgung der chemikalienbehafteten Abwässer sind erhebliche Aufwendungen erforderlich.

Überhitzer, Zwischenüberhitzer sowie zugehörige Dampfleitungen werden üblicherweise mit Dampf ausgeblasen. Dabei gilt es Bedingungen einzuhalten, die sicherstellen, dass Festkörperpartikel, die trotz Dampfausblasens in den Anlagenteilen verblieben sind, während des Anlagebetriebes vom Betriebsdampfstrom nicht mitgerissen werden.

Daher ist das Ausblasen nur mit höheren Strömungsgeschwindigkeiten effektvoll, als sie bei Volllastbetrieb der Anlage zu erwarten sind. Erfahrungsgemäß beträgt der Staudruck beim Ausblasen den 1,2- bis 1,7-fachen Wert des Staudrucks während des Betriebes bei maximaler Dauerleistung der Anlage. Diese Ausblasebedingung lässt sich sowohl bei einem kontinuierlichen Ausblasen bei relativ niedrigem Druck als auch bei einem diskontinuierlichen Ausblasen bei relativ hohem Druck erfüllen.

Beiden Verfahren - stoßartiges oder kontinuierliches Dampfausblasen - liegt zugrunde, dass der an die Atmosphäre abgegebene Dampf durch Nachspeisung von voll entsalztem Wasser (= Deionat) ersetzt werden muss. Die Dampfreinigungsdauer ist somit stets abhängig von der Deionaterzeugungsleistung bzw. Deionatspeicherkapazität. Darüber hinaus sind aufwendige Ausblaseprovisorien notwendig, um eine schnelle Deionatnachspeisung in das Kondensatsystem sicherzustellen und um die beim atmosphärischen Dampfausblasen auftretenden Schallemissionen auf die geforderten Schallpegel zu begrenzen. Beispielsweise werden hierzu Schalldämpfer verwendet bzw. erfolgt eine Wassereinspritzung in den Dampf und führt somit zu einem zusätzlichen Wasserbedarf. Auch können die herkömmlichen Kreislaufreinigungen nicht unterbrechungsfrei durchgeführt werden, da die Beurteilung der Partikelfreiheit des Dampfes in hohen Temperatur- und Druckbereichen nicht online möglich ist.

Der Erfindung liegt daher die Aufgabe zugrunde, ein gegenüber dem Stand der Technik verbessertes Verfahren zum Reinigen von Anlagenteilen einer Kraftwerksanlage anzugeben. Des Weiteren sind eine besonders einfache Vorrichtung zum Reinigen von Anlagenteilen sowie eine besonders geeignete Messeinrichtung anzugeben.

Die erstgenannte Aufgabe wird erfindungsgemäß gelöst durch den Gegenstand des Anspruchs 1. Hinsichtlich der Vorrichtung wird die Aufgabe gelöst durch die Merkmale des Anspruchs 9. Hinsichtlich der Messeinrichtung wird die Aufgabe gelöst durch die Merkmale des Anspruchs 14.

Vorteilhafte Weiterbildungen der Erfindung sind Gegenstand der Unteransprüche.

Die Erfindung geht dabei von der Überlegung aus, dass für einen möglichst unterbrechungsfreien Reinigungsprozess von insbesondere dampfführenden Anlagenteilen einer Kraftwerksanlage der Reinigungsprozess im Kreislaufbetrieb durchgeführt werden sollte. Dabei wird als Medium im Kondensator kondensierter Dampf kontinuierlich in einem geschlossenen Strömungskreis durch ein oder mehrere zu reinigende Anlagenteile geführt, wobei eine Prüfung des Mediums auf seinen Reinheitsgrad in mindestens einem betrieblichen Anlagenteil durchgeführt wird. Hierdurch ist eine Reinigung ohne Unterbrechung bis zur Erreichung der erforderlichen Partikelfreiheit ermöglicht, wodurch wiederum die Reinigungszeiten erheblich reduziert werden. Ob die erforderliche Partikelfreiheit vorliegt, wird mittels einer direkt in einem betrieblichen Anlagenteil angeordneten Messeinrichtung erfasst. Durch eine geschlossene Dampfreinigung ist der Reinigungsprozess unabhängig von der Deionaterzeugungsleistung bzw. Deionatspeicherkapazität. Auch eine schnelle Nachspeisung des Deionats ist nicht erforderlich, da der geschlossene Reinigungsprozess nahezu wasserverlustfrei durchgeführt werden kann.

Vorzugsweise wird der Reinheitsgrad des Mediums, insbesondere des Dampfes, in einer betrieblichen und somit permanenten Dampfleitung geprüft. Hierdurch können erforderliche Dampfreinigungsparameter und Reinigungsschritte während der Reinigung im betrieblichen Anlagenteil selbst geprüft und somit unterbrechungsfrei eingestellt werden. Zusätzlich oder alternativ kann der Reinheitsgrad des Mediums in einer temporären Leitung geprüft werden. Unter einer temporären Leitung wird insbesondere eine für den Reinigungsprozess erforderliche und zu installierende temporäre Dampfleitung zur Umführung der Hochdruckdampfturbine verstanden.

In einer möglichen Ausführungsform für einen Kreislaufbetrieb des Reinigungsprozesses wird der Reinheitsgrad des Mediums im Kondensationsbetrieb der Kraftwerksanlage geprüft. Aufgrund der geringen Schallpegelbelastung im Kondensationsbetrieb ist der Reinigungsprozess unabhängig von Tageszeit-, Wochenend- bzw. Feiertagsbeschränkungen und kann während des laufenden Betriebes der Kraftwerksanlage durchgeführt werden.

Zum Reinigen der Anlagenteile mittels Dampf im Kreislaufbetrieb wird dabei von einer Dampfkessel-Einrichtung Dampf entnommen und in einem Kondensator aus dem Dampf gewonnene Flüssigkeit dem oder den betrieblichen Anlagenteilen zugeführt und in einem geschlossenen Strömungskreis geführt. Im Falle einer Anlage, die eine Dampfturbinen-Einheit umfasst, wird diese vom Strömungskreis umgangen und während der Reinigung nicht mit Dampf beaufschlagt. Somit werden die empfindlichen Teile der Dampfturbine nicht durch ausgeblasene Fremdkörper oder Partikel beschädigt. Jedoch wird die Dampfturbine während des Kondensationsbetriebs hydraulisch gedreht, um eine Wellenverkrümmung - die durch Wärmestrahlung des Dampfes im Dampfraum des Kondensators hervorgerufen würde - zu vermeiden. Beim Kondensationsbetrieb befindet sich daher die Dampfturbine im so genannten Drehbetrieb, der Kondensator wird dampfseitig evakuiert und kühlwasserseitig durch Kühlwasser gekühlt.

Vorzugsweise werden als zu reinigende Anlagenteile zumindest ein Kessel oder nacheinander oder parallel mehrere Kessel in den Dampfkreislauf einbezogen und anschließend schrittweise weitere betriebliche Anlagenteile, die in der Kraftwerksanlage strömungsmäßig den bereits gereinigten Anlagenteilen nachgeschaltet sind, in den geschlossenen Strömungskreislauf geschaltet.

Mit anderen Worten: Der geschlossene Reinigungsprozess ermöglicht eine parallele Systeminbetriebsetzung von Gasturbine, Kessel und Wasser-Dampfkreislauf sowie von Umleitstationen bis zur Grundlast der Anlage. Somit kann der Zeitpunkt für den Rückbau von erforderlichen Ausblaseprovisorien vorgegeben und geplant werden. Bedingt durch die im normalen Kondensatorbetrieb erfolgte Reinigung in einem geschlossenen Kreislauf sind besonders einfache und auf wenige Komponenten reduzierte Ausblaseprovisorien ermöglicht. Der reduzierte Umfang an Ausblaseprovisorien verkürzt zudem die Rückbauzeit, die zur Herstellung des Anlagenzustands nach jeder Reinigung erforderlich ist. Als Ausblaseprovisorien ist beim vorliegenden Verfahren lediglich ein temporäres Dampfleitungssystem zur Umgehung der Hochdruckstufe der Dampfturbine vorgesehen.

Die Vorrichtung umfasst zum Reinigen von Anlagenteilen einer Kraftwerksanlage in einem geschlossenen Strömungskreis mindestens eine an einem betrieblichen Anlagenteil anschließbare Messeinrichtung zur Messung des Reinheitsgrads des Mediums (auch als "Prallplattenwechsel-Einrichtung" bezeichnet). Die Messeinrichtung umfasst dabei zur Prüfung der Dampfreinheit eine direkt in eine betriebliche Dampfleitung einführbare Messvorrichtung, die entsprechend druck- und temperaturfest ausgeführt ist. Insbesondere ist die Messvorrichtung gegenüber einem Druck von ca. 40 bar und einer Temperatur von 550 °C hinreichend fest ausgeführt.

Aufgrund der Auslegung der Messvorrichtung hinsichtlich einer vorgegebenen Druckfestigkeit eignet sich die Messeinrichtung zum Anschluss an eine Mitteldruck- bzw. Niederdruck-Dampfleitung (im Weiteren auch kurz "Mitteldruck-, "ZÜ- bzw. "Niederdruck-Dampfleitung" genannt). Hierdurch kann die Messeinrichtung in geflanschter Ausführung an einen Stutzen einer der permanenten oder betrieblichen Dampfleitungen, insbesondere an die Niederdruck-Dampfleitung und/oder an eine Mitteldruck-Dampfleitung angeschlossen werden. Auch ist es möglich, die Messeinrichtung an einen Stutzen einer temporären Leitung, z. B. an den Stutzen der temporären Dampfleitung zur Umführung der Hochdruckdampfturbine, anzuschließen.

Zur Überwachung und Prüfung der Dampfreinheit ist die an eine der Dampfleitungen angeschlossene Messeinrichtung im Kondensationsbetrieb der Kraftwerksanlage geöffnet, insbesondere zeitweise geöffnet.

Für eine druck- und temperaturfeste Auslegung der Messeinrichtung umfasst diese mindestens eine Flanschverbindung zum Anschließen an einen Stutzen der betrieblichen Dampfleitung und eine als Schleusenarmatur ausgebildete Doppelabsperrung. Die Doppelabsperrung ermöglicht eine Trennung zwischen Dampfraum der Dampfleitung und Schleusenraum und dient der sicheren Absperrung nach dem Schleusvorgang und der Druckentlastung. Zur Druckentlastung weist die Doppelabsperrung zwischen den beiden Absperrarmaturen zweckmäßigerweise ein als Entlastungsventil ausgebildetes Absperrventil auf. Die Druckentlastung zwischen den zwei Absperrarmaturen der Doppelabsperrung kann gleichzeitig als Leckagekontrolle der Erstabsperrung der beiden Absperrarmaturen dienen. Bei Bedarf kann zusätzlich an die Doppelabsperrung ein Kühlelement angeschlossen werden.

Die Flanschverbindung ist beispielsweise als eine standardmäßige 3"-Flanschverbindung (= 3-Zoll-Flanschverbindung) ausgeführt. Eine derartige Dimensionierung dient der sicheren Aufnahme eines unteren Lagers im Stutzen für die Messvorrichtung, die in das betriebliche Anlagenteil zur Prüfung des Reinheitsgrades des Dampfes einführbar ist.

In einer bevorzugten Ausführungsform ist die Doppelabsperrung eingangs- und/oder ausgangsseitig mit mindestens einem elastischen Dichtelement versehen. Die Dichtelemente sind derart elastisch ausgeführt, dass Temperaturgradienten ausgeglichen werden.

Die Flanschverbindung unterhalb der Schleuse umfasst im Bereich des Stutzens zum Dampfraum ein Auflager. Das Auflager dient der Aufnahme der Messvorrichtung, die an einem Spindelende befestigt ist. Hierdurch ist die Einschleusung der Messvorrichtung in die Dampfleitung und somit in eine vibrierende Strömung unproblematisch und muss nicht zeitlich begrenzt werden. Vorzugsweise ist das Auflager als eine Hülse mit einem umlaufenden Rand oder einem Konus ausgebildet. Dazu weist das Auflager zumindest teilweise einen angefasten Innenabsatz, insbesondere mit einem Winkel von größer 18°, auf. Der angefaste oder schräge Innenabsatz dient der einfachen Aufnahme und Halterung. Die Messvorrichtung, die korrespondierend zum Fasenwinkel des Innenabsatzes im Bereich der Befestigung ebenfalls angefast ausgebildet ist, wird hierdurch besonders sicher gelagert.

Um ein Hineinfallen von losen Teilen, z. B. Befestigungsschrauben, in den Dampfraum zu vermeiden, ist die Befestigung der Messvorrichtung im eingeschleusten Zustand gekapselt ausgeführt. Darüber hinaus kann mittels der Befestigung auch die Einfahrtiefe der Messvorrichtung in die Dampfleitung eingestellt werden. Zum Ein- und Ausführen der Messvorrichtung in die Dampfleitung ist ein mechanischer Antrieb, insbesondere eine Gewindespindel mit Mutter, vorgesehen. Die.Gewindespindel, die maschinell über einen Motor oder manuell über ein Handrad betätigbar ist, ist dehnungskompensiert und temperaturkompensiert ausgeführt und von einem Gehäuse umgeben. Das Spindelende führt als Drehspindel aus dem Gehäuse, wo der Antrieb befestigt ist. Die Anordnung der Spindelmutter als auch deren Kompensation gegen Dehnungen und Verschiebungen verhindert eine sonst übliche Schwergängigkeit durch Schmutzeintrag oder hohe Temperaturen.

Beim Ein- und Ausführen und somit beim Wechsel der Messvorrichtung wird das oberhalb der Doppelabsperrung oder Schleusenarmatur befindliche Gehäuse vor dem Öffnen zur Entnahme der Messvorrichtung gezielt druckentlastet. Das Öffnen des Gehäuses geschieht anschließend über eine im Bereich oberhalb der Doppelabsperrung vorgesehene weitere Flanschverbindung. Die Flanschverbindung ist mit einer Dichtung, die ein leichtes Öffnen und Verschließen zulässt, versehen. Sowohl unterhalb der Flanschverbindung als auch im oberen Gehäuseteil ist ein weiteres Entlastungsventil zur Druckentlastung des Gehäuses vorgesehen. Darüber hinaus kann gezielt entlastet, gekühlt und die Dichtheit der Doppelabsperrung überwacht werden.

Für eine einfache Anordnung der Messeinrichtung an der Dampfleitung ist eine Halterung vorgesehen, die insbesondere als eine Sattelhalterung auf der Dampfleitung selber ausgebildet ist. Damit ist eine steife Verbindung zur rohrförmigen Dampfleitung hergestellt, so dass Relativbewegungen vermieden sind. Damit wird eine hinreichend sichere Befestigung auch für einen Dauereinsatz der Messeinrichtung an der Dampfleitung ermöglicht.

Die mit der Erfindung erzielten Vorteile bestehen insbesondere darin, dass durch das Dampfreinigen im Kreislaufbetrieb bei der Kondensation des Dampfes im Kondensator und somit im Kondensationsbetrieb der Anlage eine Prüfung des Reinheitsgrades des Dampfes in der betrieblichen Dampfleitung ermöglicht ist und eine Reinigung nahezu wasserverlustfrei durchgeführt werden kann. Somit ist der Reinigungsprozess nahezu unabhängig von der Deionaterzeugungsleistung-, -speicherkapazität und -nachspeisung ermöglicht. Durch die Reinigung im Kreislaufbetrieb kann aufgrund des äußerst geräuscharmen Prozesses auf aufwendige Schallschutzmaßnahmen verzichtet werden. Zudem sind nur einfache und wenige Ausblaseprovisorien erforderlich, so dass sich die Rückbauzeit, die zur Herstellung des normalen betrieblichen Anlagenzustands erforderlich ist, deutlich reduziert.

Ein Ausführungsbeispiel der Erfindung wird anhand einer Zeichnung näher erläutert. Darin zeigen:
- FIG 1: eine grobe und schematische Darstellung der für den Reinigungsprozess vorgesehenen Anlagenteile einer Kraftwerksanlage mit einer Dampfkessel-Einrichtung und einer Dampfturbinen-Einrichtung sowie die für das erfindungsgemäße Verfahren vorgesehene Messeinrichtung und die vorgesehenen Ausblaseprovisorien,
- FIG 2: eine detaillierte Darstellung einer an ein Anlagenteil angeschlossenen Messeinrichtung zur Messung des Reinheitsgrads des Mediums,
- FIG 3 bis 9: in einer detaillierten Darstellung Komponenten der Messeinrichtung, und
- FIG 10 und 11: eine detaillierte Darstellung einer Kraftwerksanlage mit mehreren während des Reinigungsprozesses durchzublasenden Anlagenteilen.

Gleiche Teile sind in allen Figuren mit denselben Bezugszeichen versehen.

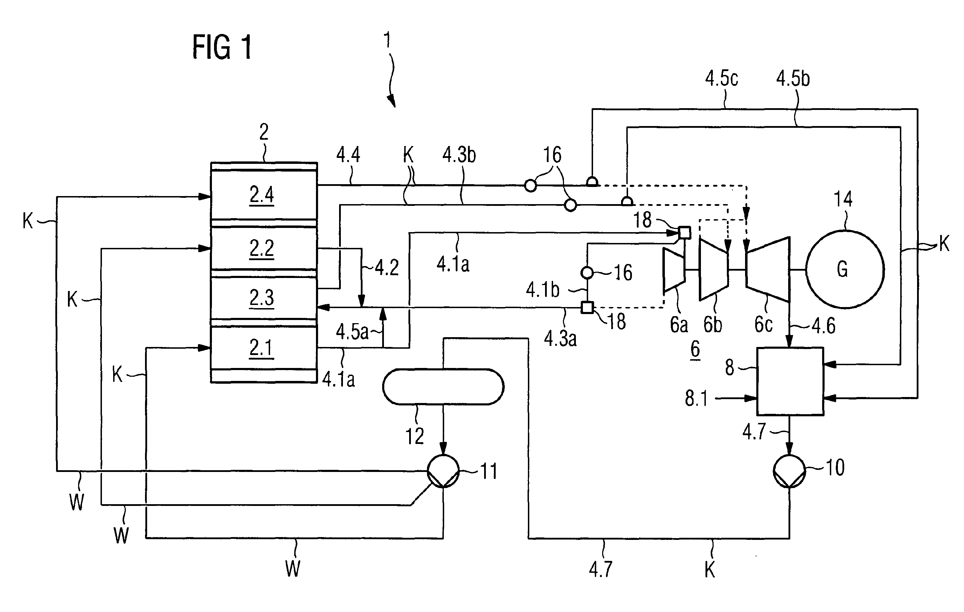

FIG 1 zeigt einen geschlossenen Strömungskreislauf K zum Reinigen der Anlagenteile einer Kraftwerksanlage 1. Der geschlossene Strömungskreislauf K ist durch eine Doppellinie gekennzeichnet.

Die Kraftwerksanlage 1 umfasst eine Dampfkessel-Einrichtung 2 mit mehreren Druckstufen 2.1 bis 2.4, z. B. bestehend aus einem Hochdruck-Teil, einem Mitteldruck-Teil, einem Niederdruck-Teil und einem Zwischenüberhitzer, die über Dampfleitungen 4.1a, 4.3b und 4.4 im Normalbetrieb der Kraftwerksanlage 1 eine Dampfturbinen-Einheit 6 speisen. Die im Reinigungsprozess umgangenen Komponenten der Kraftwerksanlage 1 sind durch eine gestrichelte Linie gekennzeichnet.

Von der Dampfturbinen-Einheit 6 geht im Normalbetrieb eine Dampfleitung 4.6 zu einer Rückspeiseeinrichtung, die einen Kondensator 8 und eine Kondensatpumpe 10 umfasst, um das aus dem kondensierten Dampf gewonnene Wasser über eine Rücklaufleitung 4.7 wieder der Dampfkessel-Einrichtung 2 zuzuführen. Wasserverluste werden über die Zuführleitung 8.1 ausgeglichen und über den Kondensator 8 und die Rücklaufleitung 4.7 dem Speisewasserbehälter 12 zugefüllt. Zur Nachspeisung der Dampfkesseleinrichtung 2 steht das Wasser im Speisewasserbehälter 12 gespeichert zur Verfügung. Mit der Dampfturbinen-Einheit 6 wird im Normalbetrieb der Kraftwerksanlage 1 ein Generator 14 zur Erzeugung elektrischen Stroms angetrieben.

Zur Befreiung der dampfführenden Anlagenteile der Dampfkesseleinrichtung 2.1 bis 2.4, 4.1a, 4.2, 4.3a, 4.3b, 4.4 sowie der betrieblichen Bypassleitungen 4.5a, 4.5b und 4.5c von Partikeln werden diese unter Umgehung der Dampfturbinen-Einheit 6 in dem geschlossenen Strömungskreislauf K von Dampf als Spülmedium durchströmt. Die umgangene Dampfturbinen-Einheit 6 und andere umgangene Komponenten, wie der Generator 14, sind gestrichelt dargestellt. Zur Umgehung der Dampfturbinen-Einheit 6 geht von den Dampfleitungen 4.3b und 4.4 jeweils eine Dampf-Bypassleitung 4.5b und 4.5c ab, die in den Kondensator 8 münden.

Die im Kondensationsbetrieb umgangene Dampfleitung 4.6, d. h. die Niederdruck-Turbinenabdampfleitung, zum Kondensator 8 wird erst nach erfolgter Dampfreinigung mit Dampf beaufschlagt, d. h. wenn die Dampfturbinen-Einheit 6 mit Dampf angestoßen wird. Die Rücklaufleitung 4.7 ist eine Kondensatleitung, die bereits im vorgeschalteten Reinigungsprozess beim Spülen gereinigt wird.

Die Reinigung der dampfführenden Anlagenteile 2.1 bis 2.4 der Dampfkesseleinrichtung 2 und der Dampfleitungen 4.1a, 4.2, 4.3a, 4.3b, 4.4, 4.5a bis 4.5c erfolgt während des Kondensationsbetriebs der Anlage 1. Dabei wird wasserseitig als Spülmedium gereinigtes und entsalztes Wasser, insbesondere Deionat, benutzt, das über die Zuführleitung 8.1 dem Kondensatsystem 4.7 zugeführt wird und ggf. im Speisewasserbehälter 12 gespeichert zur Verfügung steht. Aus diesem Spülmedium wird in den Kesseldruckstufen 2.1, 2.2 und 2.4 Dampf erzeugt. Zur Überwachung des Reinheitsgrades des Dampfes ist an mindestens einem der Anlagenteile 4.1b, 4.3b und 4.4 eine Messeinrichtung 16 zur Messung des Reinheitsgrads des Mediums (im Weiteren kurz "Messeinrichtung 16" genannt) vorgesehen. Die Messeinrichtung 16 ist als eine Prallplattenwechsel-Einrichtung ausgeführt.

Die Messeinrichtung 16 ist besonders für den Einbau in die temporäre Dampfleitung 4.1b und für die betrieblichen Dampfleitungen 4.3b, 4.4, insbesondere eine Mitteldruck- bzw. Niederdruck-Dampfleitung, entsprechend druck- und temperaturfest ausgeführt.

Bei einer eine Hochdruckstufe aufweisenden Kraftwerksanlage 1 ist von der Hockdruck-Dampfleitung 4.1a zur Umgehung der Hochdruck-Stufe der Dampfturbinen-Einheit 6 ein Abzweig für temporäre Reinigungsprovisorien mit einem temporären Dampfleitungssystem 4.1b und Ausblaseeinsätze 18 sowie einer zwischen den Ausblaseeinsätzen 18 angeordneten Messeinrichtung 16 vorgesehen.

Beim Ausblasen der dampfführenden Komponenten der Kesseldruckstufen 2.1 bis 2.4 und der Dampfleitungen 4.1a, 4.2, 4.3a, 4.3b, 4.4, 4.5a bis 4.5c der Kraftwerksanlage 1 mittels Dampf, insbesondere höchstreinem Dampf, wird lediglich der im temporären Dampfleitungssystem 4.1b zur Umgehung der Hochdruckstufe der Dampfturbineneinheit 6 geführte Dampf über die Ausblaseeinsätze 18 ausgeblasen.

Im Normalbetrieb der Kraftwerksanlage 1 mündet die Hochdruck-Dampfleitung 4.1a in die Hochdruck-Stufe 6a der Dampfturbinen-Einheit 6.

Im Folgenden wird der geschlossene Strömungskreis K zur Reinigung der Anlagenteile 4.1a, 4.2, 4.3a, 4.3b, 4.4, 4.5a bis 4.5c und der Dampfkessel-Einrichtung 2 mit den einzelnen Druckstufen 2.1 bis 2.4 näher beschrieben.

Die Reinigung der Anlagenteile 4.1a, 4.2, 4.3a, 4.3b, 4.4, 4.5a bis 4.5c und der Dampfkessel-Einrichtung 2 mit den einzelnen dampfführenden Druckstufen 2.1 bis 2.4 kann jeweils nacheinander erfolgen. Das heißt, zunächst werden der Hochdruck- und der Mitteldruck-Teil der Dampfkessel-Einrichtung 2 gereinigt. Dazu wird der Dampf aus der Hochdruckstufe der Dampfkesseleinrichtung 2 über das temporäre Dampfleitungssystem 4.1b geführt.

Beim Durchblasen der Anlagenteile der Hochdruck-Stufe werden die vom Dampf mitgenommenen Partikel über die Ausblaseeinsätze 18 in dem temporären Dampfleitungssystem 4.1b über den Zwischenüberhitzer 2.3 und über die Mitteldruck-Umleitstation im Mitteldruck-Bypass 4.5b abgeführt.

Der Zwischenüberhitzer 2.3 und der Niederdruck-Überhitzer der Kesseldruckstufe 2.4 werden hierbei auch schon mit Dampf durchströmt, jedoch werden die erforderlichen Dampfparameter für die Reinigung dieser Bereiche noch nicht erreicht. Beim Dampfreinigen des Zwischenüberhitzers 2.3 und des Niederdruck-Überhitzers der Kesseldruckstufe 2.4 wird der Dampf über die betriebliche Hochdruck-Bypassleitung 4.5a geführt; das temporäre Dampfleitungssystem 4.1b ist entweder bereits entfernt oder es wird umgangen und somit nicht durchströmt. In diesem Schritt wird die Gasturbinenleistung soweit angehoben, dass die Reinigungsparameter für den Zwischenüberhitzer 2.3 und den Niederdruck-Überhitzer der Kesseldruckstufe 2.4 erreicht werden.

Die Qualität des Dampfes hinsichtlich seiner Partikel- oder Fremdkörperfreiheit wird mittels der Messeinrichtung 16 an entsprechender Stelle direkt in einem der betrieblichen Anlagenteile 4.3b und 4.4 bzw. beim Hochdruck-Systemreinigen im temporären Dampfleitungssystem 4.1b erfasst und während des Reinigungsvorgangs überwacht.

Anstelle eines Dampfkessels 2 kann die Dampfturbine 6 auch von mehreren Dampfkesseln 2 und 3 gespeist werden. In diesem Fall ist es möglich, dass die Dampfkessel 2 und 3, die im Normalbetrieb parallel geschaltet sind, auch im Spülmedium-Kreislauf parallel zu schalten und somit gleichzeitig zu reinigen sind. Hierdurch kann die Gesamtreinigungsdauer weiter verkürzt werden.

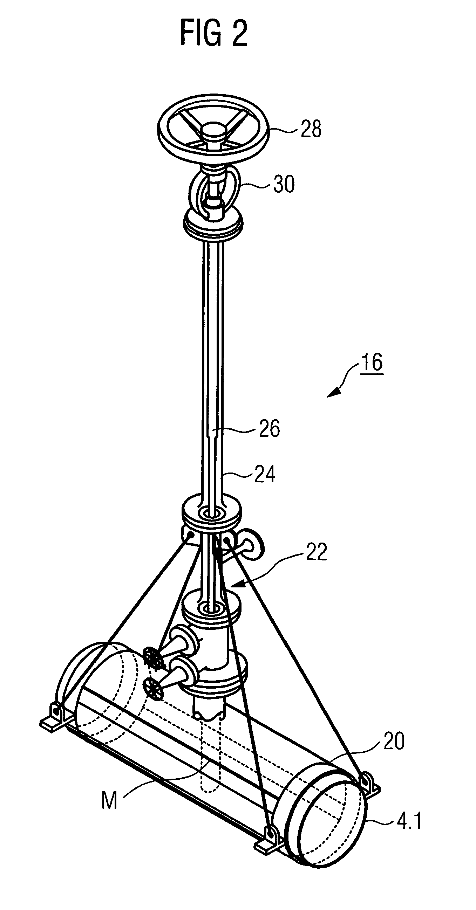

Nachfolgend wird die Messeinrichtung 16 anhand der FIG 2 bis 9 näher beschrieben.

FIG 2 zeigt eine beispielsweise an eine Dampfleitung 4.1, z. B. die Hochdruck-Dampfleitung 4.1a oder das temporäre Dampfleitungssystem 4.1b, angeschlossene Messeinrichtung 16. Über eine als Sattelhalterung ausgeführte Halterung 20 ist die Messeinrichtung 16 starr an der rohrförmigen Dampfleitung 4.1 befestigt, so dass Relativbewegungen vermieden sind.

Mittels der Messeinrichtung 16 wird im Inneren der Dampfleitung 4.1 im laufenden Betrieb als Messvorrichtung M ein so genannter Ausblasespiegel (auch "Prallplatte" genannt) eingeführt. Dabei handelt es sich um einen polierten Metallstreifen, z. B. einen Aluminium-, Kupfer- oder Stahlstreifen. Die Messvorrichtung M wird dabei quer zur Strömungsrichtung in die Dampfleitung 4.1 eingeführt. Der Reinheitsgrad des Mediums wird während des Ausblasevorgangs durch auf die Messvorrichtung M auftreffende Partikeleinschläge optisch beobachtet und beurteilt.

Die Messvorrichtung M ist als eine Prallplattenwechsel-Einrichtung ausgeführt, so dass sie während des Betriebes, d. h. während des Ausblasens, und des Reinigungsvorgangs im laufenden Kondensationsbetrieb gewechselt werden kann.

Um ein Auswechseln während des Ausblasens zu ermöglichen, ist eine Doppelabsperrung 22 vorgesehen, die als eine Schleusenarmatur ausgebildet ist. Oberhalb der Doppelabsperrung 22 ist ein rohrförmiges Gehäuse 24 mit einer Gewindespindel 26 angeordnet. Die Gewindespindel 26 dient dem Ein- und Ausführen der Messvorrichtung M in den Dampfraum der Dampfleitung 4.1. Das Ein- bzw. Ausführen kann manuell über ein oberhalb des Gehäuses 24 vorgesehenes Handrad 28 oder über einen nicht näher dargestellten motorisch betriebenen Antrieb erfolgen. Auch kann ein Antrieb 30 mit Stopfbuchse und zentraler Tellerfederanpressung vorgesehen sein.

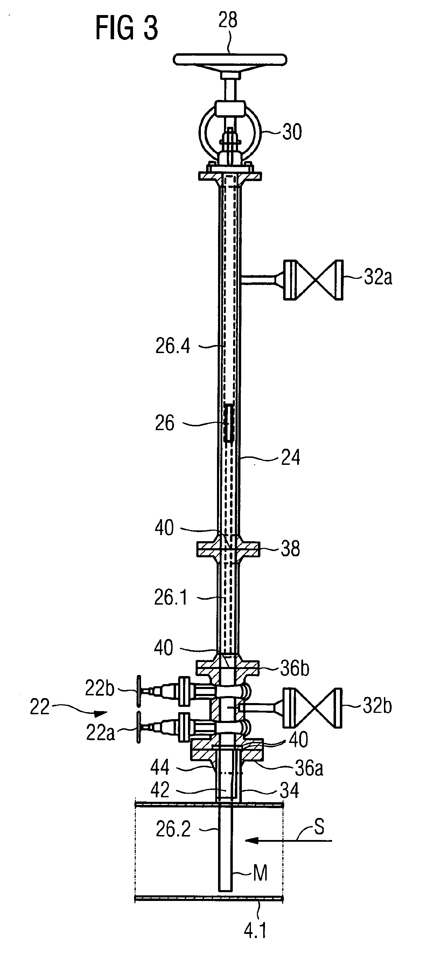

Das Gehäuse 24 kann gezielt druckentlastet werden. Hierzu umfasst die Messeinrichtung 16 zwei Entlastungsventile 32a, 32b, z. B. Absperrventile, die in der FIG 3 näher dargestellt sind. FIG 3 zeigt die Messeinrichtung 16 im Detail.

Eines der Absperrventile 32a ist oberhalb der Doppelabsperrung 22 am Gehäuse 24 zur Druckentlastung des Gehäuses 24 angeordnet. Das andere Absperrventil 32b ist zwischen den Absperrarmaturen 22a, 22b der Doppelabsperrung 22 zur Druckentlastung des Schleusenraums angeordnet. Hierzu ist die Doppelabsperrung 22 als eine Einheit oder als eine Blockarmatur aus zwei Absperrarmaturen 22a, 22b als Drehteller ausgeführt. Die mittels des betreffenden Entlastungsventils 32b bewirkte Druckentlastung zwischen den beiden Absperrarmaturen 22a, 22b dient gleichzeitig als Leckagekontrolle für die Erstabsperrung 22a.

Die Messeinrichtung 16 ist an einen Stutzen 34 der Dampfleitung 4.1 über eine Flanschverbindung 36a der Doppelabsperrung 22 angeschlossen. Am anderen Ende der Doppelabsperrung 22 ist eine weitere Flanschverbindung 36b zum Anschließen des Gehäuses 24 gegebenenfalls über einen Zwischenflansch 38 vorgesehen.

Eingangs- und ausgangsseitig der Doppelabsperrung 22 sind im Bereich der Flanschverbindungen 36a, 36b und gegebenenfalls im Bereich des Zwischenflansches 38 elastische Dichtelemente 40 vorgesehen, um Temperaturgradienten aufzufangen.

Die Flanschverbindung 36a am Stutzen 34 ist als eine 3"-Flanschverbindung ausgeführt. Mit dieser Dimensionierung wird eine Ausbildung eines unteren Lagers 42 im Stutzen 34 möglich. Das untere Lager 42 umfasst ein im Stutzen 34 zum Dampfraum eingebautes Auflager 44, das unterhalb der Schleusenarmatur 22 angeordnet ist. Das untere Lager 42 nimmt das Spindelende der Gewindespindel 26 auf, an dem die Messvorrichtung M befestigt ist, die quer zur Strömungsrichtung S in die Dampfleitung 4.1 einführbar ist.

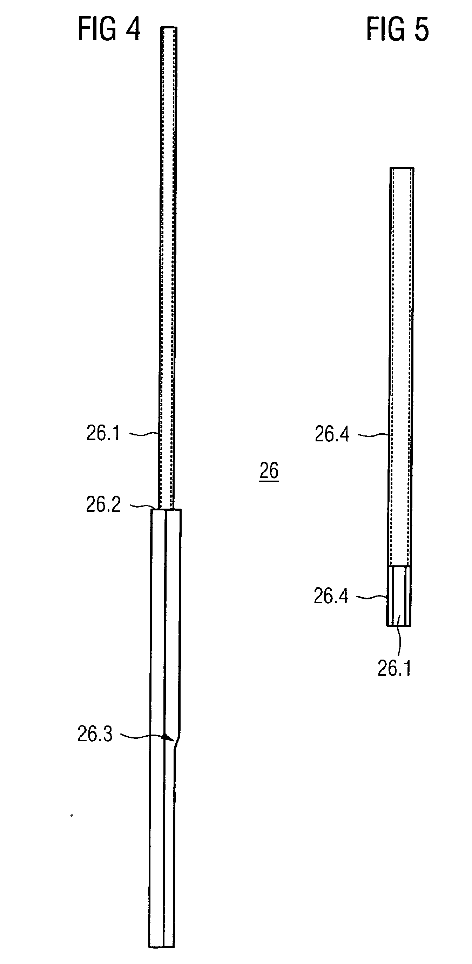

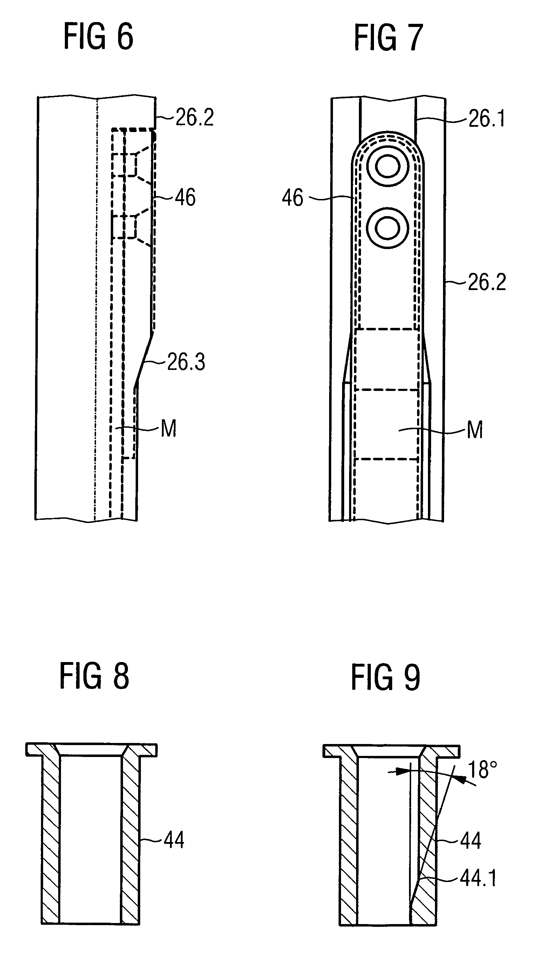

FIG 4 zeigt im Detail die Gewindespindel 26, die aus einer Gewindestange 26.1 beispielsweise mit einem Trapezaußengewinde und einem unteren Spindelgehäuse 26.2 zur Aufnahme der Messvorrichtung M gebildet ist. Das untere Spindelgehäuse 26.2 ist mit einem Innengewinde zum Führen der Gewindestange 26.1 versehen. Das untere Spindelgehäuse 26.2 weist einen nach innen angefasten Absatz 26.3 auf, wobei oberhalb des Absatzes 26.3 ein Raumbereich für eine Befestigung der Messvorrichtung M gebildet ist. Unterhalb des Absatzes 26.3 dient das Spindelgehäuse 26.2 zur Aufnahme der Messvorrichtung M. FIG 5 zeigt das obere Spindelgehäuse 26.4, das als Gewindebuchse mit einem Trapezinnengewinde zum Ein- und Ausführen der Gewindestange 26.1 vorgesehen ist.

Die FIG 6 und FIG 7 zeigen eine mögliche Ausführungsform für eine Befestigung 46 der Messvorrichtung M an der Gewindestange 26.1. Die Befestigung 46 ist als eine Doppelschraube mit einem angefasten Absatz ausgebildet, der korrespondierend zum Absatz 26.3 des unteren Spindelgehäuses 26.2 ausgebildet und von diesem aufgenommen wird, insbesondere auf diesem aufliegt. Hierdurch ist die Befestigung 46 der Messvorrichtung M im eingeschleusten Zustand gekapselt, so dass keine Befestigungsschrauben oder lose Teile in den Dampfraum hineinfallen können.

FIG 8 und FIG 9 zeigen das Auflager 44 zur Aufnahme des Spindelendes, d. h. des unteren Spindelgehäuses 26.2, im Detail. Zur Aufnahme, insbesondere zum Aufliegen, der Befestigung 46 der Messvorrichtung M weist das Auflager 44 einen mit dem Absatz 26.3 des Spindelgehäuses 26.2 korrespondierenden Absatz 44.1, insbesondere einen angefasten Absatz beispielsweise mit einem Winkel von ca. 18°, auf. Das Auflager 44 ist als eine Hülse mit einem umlaufenden Rand R ausgebildet, der in der Flanschverbindung 36a gehalten ist.

Nachfolgend wird das Verfahren zum Auswechseln der Messvorrichtung M näher beschrieben.

Bei je nach Ausführungsform manueller oder motorisch angetriebener Gewindespindel 26 wird beispielsweise bei einem manuellen Antrieb das Handrad 28 bis zum Anschlag nach oben gedreht. Anschließend wird die untere Absperrarmatur 22a der Doppelabsperrung 22 geschlossen. Die Entlastungsventile 32a und 32b werden so lange geöffnet, bis kein Dampf mehr kommt. Zur zusätzlichen Sicherheit wird die obere Absperrarmatur 22b der Doppelabsperrung 22 geschlossen.

Nach Abkühlung wird die obere Schraubenreihe des Zwischenflansches 38 geöffnet und die Gewindespindel 26 mit dem Gehäuse 24 nach oben gezogen und zum Auswechseln der Messvorrichtung M mit der Messprobe entsprechend befestigt.

Das Handrad 28 wird nach unten gedreht, bis die Befestigung 46 der Messvorrichtung M sichtbar wird. Die Messvorrichtung M kann anschließend nach Lösen der Befestigung 46 ausgetauscht werden. Nach Anbringen einer neuen Messvorrichtung M wird das Handrad 28 nach oben gedreht. Die Gewindespindel 26 wird mit dem Gehäuse 24 wieder auf den Zwischenflansch 38 aufgesetzt, wobei eine genaue Position für einen guten Sitz des Dichtelements 40 von Bedeutung ist. Der Zwischenflansch 38 wird dann geschlossen. Auch die Entlastungsventile 32a, 32b werden geschlossen. Die Doppelabsperrung 22, d. h. beide Absperrarmaturen 22a, 22b, werden geöffnet und das Handrad 28 wird bis zum Anschlag nach unten gedreht, wodurch die Messvorrichtung M in die Dampfleitung 4.1 geführt wird. Dabei wird das Handrad 28 mit Gefühl betätigt, d. h., so weit in den Konus gedreht, dass keine Vibration auftritt.

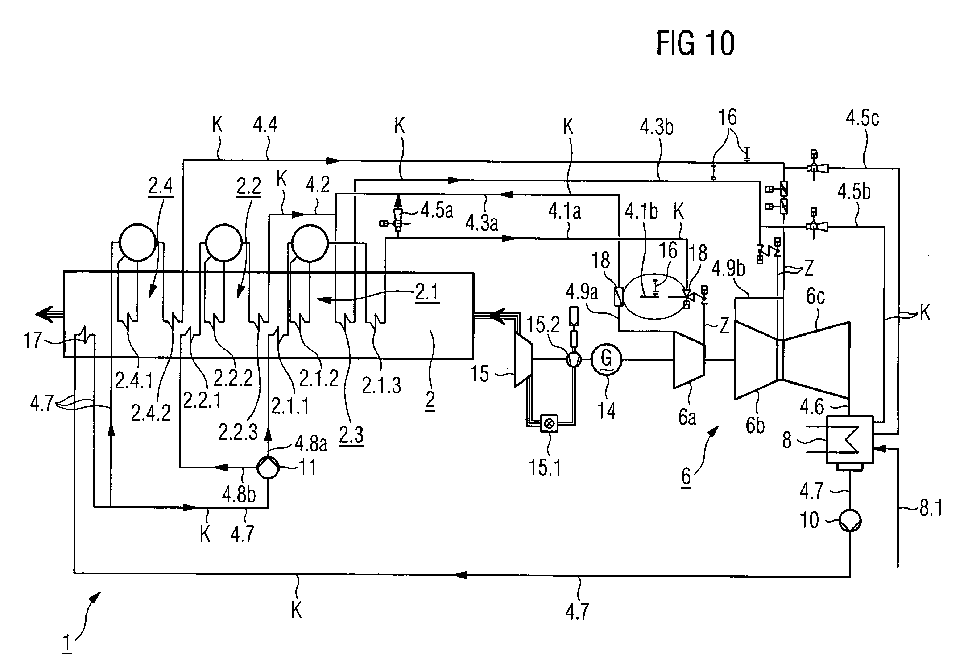

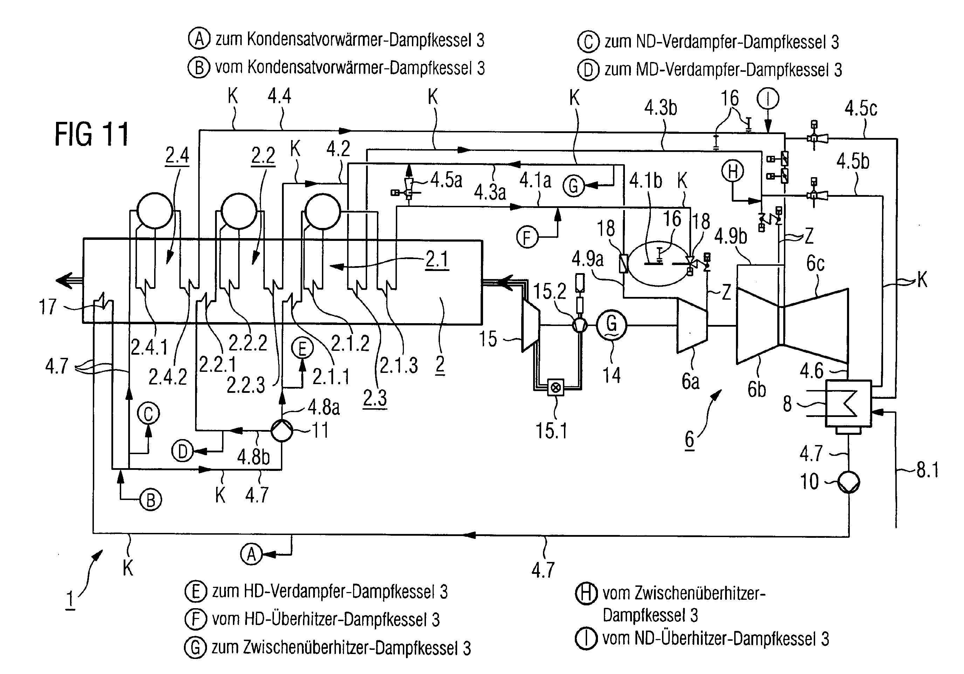

Die FIG 10 und 11 zeigen zwei verschiedene Ausführungsbeispiele für einen geschlossenen Strömungskreislauf K zum Reinigen der dampfführenden Anlagenteile der Kesseldruckstufen 2.1 bis 2.4 und der Dampfleitungen 4.1a, 4.2, 4.3a, 4.3b, 4.4, 4.5a bis 4.5c einer Kraftwerksanlage 1 mit einer oder mehreren Dampfkessel-Einrichtungen 2 bzw. 2 und 3.

Die FIG 10 zeigt beispielsweise eine herkömmliche Gas- und Dampfturbinenanlage mit einer 3-stufigen Dampfturbineneinheit 6, einer Gasturbine 15 und einer einzelnen Dampfkesseleinrichtung 2 mit mehreren Druckstufen mit einer Hochdruck-Stufe 2.1 aus Hochdruck-Economizer 2.1.1, Hochdruck-Verdampfer 2.1.2 und Hochdruck-Überhitzer 2.1.3, einer Mitteldruck-Stufe 2.2 aus Mitteldruck-Economizer 2.2.1, Mitteldruck-Verdampfer 2.2.2 und Mitteldruck-Überhitzer 2.2.3, einem Zwischenüberhitzer 2.3 und einer Niederdruck-Stufe 2.4 aus Niederdruck-Verdampfer 2.4.1 und Niederdruck-Überhitzer 2.4.2. Den Druckstufen 2.1 bis 2.4 der Dampfkessel-Einrichtung 2 ist ein Kondensatvorwärmer 17 vorgeschaltet.

In der FIG 10 ist ein Hochdruck-Trommelkessl gezeigt. Anstelle des Hochdruck-Trommelkessels kann auch ein Zwangdurchlaufdampferzeuger installiert sein. Gegebenenfalls kann zusätzlich zur Speisewasserpumpe 11 ein Speisewasserbehälter oder ein Entgaser in der Kondensatleitung 4.7 angeordnet sein. Die Kraftwerksanlage 1 ist eine Einwellenanlage, bei welcher ein Generator gemeinsam für die Gasturbine und die Dampfturbine vorgesehen ist. Gleiches gilt für die FIG 11.

In der FIG 10 ist der geschlossene Strömungskreislauf K zum Reinigen der betrieblichen Anlagenteile.2.1.3, 4.1a, 2.2.3, 4.2, 4.3a, 4.3b, 2.4.2, 4.4, 4.5a bis 4.5c der Kraftwerksanlage 1 mit einer Doppellinie dargestellt. Die mit einer dicken Volllinie dargestellten Komponenten 4.6, 4.9a, 4.9b der Dampfturbinen-Einheit 6 werden hierbei umgangen. Die Zuleitungen Z sind durch Armaturen geschlossen, so dass diese sowie die Dampfturbinen-Einheit 6 ebenfalls umgangen werden und nicht von Dampf durchströmt werden; die Dampfturbinen-Einheit 6 wird lediglich hydraulisch gedreht, um eine Wellenverkrümmung zu vermeiden.

Zur Reinigung der Komponenten durch Dampf werden die betreffenden Komponenten, d. h. die Dampfleitungen 4.1a, 4.2, 4.3a, 4.3b, 4.4 und das temporäre Dampfleitungssystem 4.1b sowie die betriebliche Bypassleitungen 4.5a bis 4.5c und die Dampfkessel-Einrichtung 2 mit den Überhitzerheizflächen 2.1.3, 2.2.3, 2.3, 2.4.2 im Kondensationsbetrieb der Kraftwerksanlage 1 ausgeblasen. Dabei werden zunächst der Hochdruck- und der Mitteldruck-Überhitzer 2.1.3 und 2.2.3 der Dampfkessel-Einrichtung 2 durch Einstellung geeigneter Dampfparameter gereinigt. Der Zwischenüberhitzer 2.3 und der Niederdruck-Überhitzer 2.4.2 werden in diesem Zyklus auch schon mit Dampf durchströmt. Durch Erhöhung der Leistung einer der Dampfkessel-Einrichtung 2 vorgeschalteten Gasturbine 15 werden anschließend die Reinigungsparameter für den Zwischenüberhitzer 2.3 und den Niederdruck-Überhitzer 2.4.2 eingestellt. Der Gasturbine 15 sind eine Brennkammer 15.1 und ein Verdichter 15.2 zum Betreiben der Gasturbine 15 vorgeschaltet.

Zwischen die temporären Ausblaseeinsätze 18 ist das temporäre Dampfleitungssystem 4.1b zur Umgehung der Hochdruck-Stufe 6a der Dampfturbine 6 angeordnet und mit einer Messeinrichtung 16 versehen. Mittels der Messeinrichtung 16, z. B. einer Prallplattenwechsel-Einrichtung, werden die im Dampf mitgeführten Partikel gemessen. Das Abführen der mitgenommenen Partikel oder Fremdkörper erfolgt über die Ausblaseeinsätze 18 durch Ausblasen des Dampfes. Die Deionatnachspeisung erfolgt dabei über eine Zuführungsleitung 8.1, die in den Kondensator 8 mündet. Die Messeinrichtung 16 ist in den Dampfleitungen 4.4 und 4.3b der Niederdruck-Stufe 2.4 bzw. der Mitteldruck-Stufe 2.2 und in dem temporären Dampfleitungssystem 4.1b angeordnet.

Im Unterschied zur FIG 10 ist in der FIG 11 eine Kraftwerksanlage 1 mit mehreren Dampfkessel-Einrichtungen 2, 3 dargestellt. Die weitere Dampfkessel-Einrichtung 3 wird gemäß den Zu- bzw. Abführungen A bis I mit Wasser bzw. mit Dampf durchströmt.

## Patentansprüche

1. Verfahren zum Reinigen von Anlagenteilen (2.1.3, 2.2.3, 2.3, 2.4.2, 4.1a, 4.2, 4.3a, 4.3b, 4.4, 4.5a bis 4.5c) einer Kraftwerksanlage (1), wobei ein Medium kontinuierlich in einem geschlossenen Strömungskreis (K) durch ein oder mehrere zu reinigende Anlagenteile (2.1.3, 2.2.3, 2.3, 2.4.2, 4.1a, 4.2, 4.3a, 4.3b, 4.4, 4.5a bis 4.5c) geführt wird und eine Prüfung des Mediums auf seinen Reinheitsgrad in mindestens einem betrieblichen Anlagenteil (4.1a, 4.2, 4.3a, 4.3b, 4.4, 4.5a bis 4.5c) durchgeführt wird.

2. Verfahren nach Anspruch 1, wobei der Reinheitsgrad des Mediums in einer Dampfleitung (4.1a, 4.2, 4.3a, 4.3b, 4.4, 4.5a bis 4.5c) geprüft wird.

3. Verfahren nach Anspruch 1 oder 2, wobei der Reinheitsgrad des Mediums in einem temporären Dampfleitungssystem (4.1b) geprüft wird.

4. Verfahren nach einem der Ansprüche 1 bis 3, wobei der Reinheitsgrad des Mediums beim Kondensationsbetrieb der Kraftwerksanlage (1) geprüft wird.

5. Verfahren nach einem der Ansprüche 1 bis 4, wobei als Medium Dampf durch die betrieblichen Anlagenteile (2.1.3, 2.2.3, 2.3, 2.4.2, 4.1a, 4.2, 4.3a, 4.3b, 4.4, 4.5a bis 4.5c) geführt wird.

6. Verfahren nach einem der Ansprüche 1 bis 5, wobei zum Reinigen der Anlagenteile (2.1.3, 2.2.3, 2.3, 2.4.2, 4.1a, 4.2, 4.3a, 4.3b, 4.4, 4.5a bis 4.5c) mittels Dampf von einer Dampfkessel-Einrichtung (2) Dampf entnommen und in einem Kondensator (8) aus dem Dampf gewonnene Flüssigkeit dem oder den betrieblichen Anlagenteilen (2.1.3, 2.2.3, 2.3, 2.4.2, 4.1a, 4.2, 4.3a, 4.3b, 4.4, 4.5a bis 4.5c) zugeführt und in einem geschlossenen Strömungskreis (K) geführt wird.

7. Verfahren nach einem der Ansprüche 1 bis 6, wobei vom Strömungskreis (K) eine in der Kraftwerksanlage (1) vorgesehene Dampfturbinen-Einheit (6) umgangen wird und die Dampfturbinen-Einheit (6) während der Reinigung nicht mit Dampf beaufschlagt wird.

8. Verfahren nach einem der Ansprüche 1 bis 7, wobei als zu reinigende Anlagenteile (2.1.3, 2.2.3, 2.3, 2.4.2, 4.1a, 4.2, 4.3a, 4.3b, 4.4, 4.5a bis 4.5c) zumindest ein Kessel (2) oder nacheinander oder parallel mehrere Kessel (2, 3) in den Dampfkreislauf einbezogen werden und anschließend schrittweise weitere betriebliche Anlagenteile (2.1.3, 2.2.3, 2.3, 2.4.2, 4.1a, 4.2, 4.3a, 4.3b, 4.4, 4.5a bis 4.5c), die in der Kraftwerksanlage (1) strömungsmäßig den bereits gereinigten Anlagenteilen (2.1.3, 2.2.3, 2.3, 2.4.2, 4.1a, 4.2, 4.3a, 4.3b, 4.4, 4.5a bis 4.5c) nachgeschaltet sind, in den geschlossenen Strömungskreislauf (K) geschaltet werden.

9. Vorrichtung zum Reinigen von Anlagenteilen (2.1.3, 2.2.3, 2.3, 2.4.2, 4.1a, 4.2, 4.3a, 4.3b, 4.4, 4.5a bis 4.5c) einer Kraftwerksanlage (1), wobei ein Medium kontinuierlich in einem geschlossenen Strömungskreis (K) durch ein oder mehrere zu reinigende Anlagenteile (2.1.3, 2.2.3, 2.3, 2.4.2, 4.1a, 4.2, 4.3a, 4.3b, 4.4, 4.5a bis 4.5c) führbar ist und an mindestens einem betrieblichen Anlagenteil (4.1a, 4.2, 4.3a, 4.3b, 4.4, 4.5a bis 4.5c) eine Messeinrichtung (16) zur Messung des Reinheitsgrads des Mediums anschließbar ist.

10. Vorrichtung nach Anspruch 9, wobei die Messeinrichtung (16) an einen Stutzen (34) einer Dampfleitung (4.1a, 4.2, 4.3a, 4.3b, 4.4, 4.5a bis 4.5c), insbesondere an eine Niederdruck-Dampfleitung und/oder an eine Mitteldruck-/ZÜ-Dampfleitung, anschließbar ist.

11. Vorrichtung nach Anspruch 9 oder 10, wobei die Messeinrichtung (16) an einen Stutzen (34) eines temporären Dampfleitungssystems (4.1b) anschließbar ist.

12. Vorrichtung nach einem der Ansprüche 9 bis 11, wobei die Messeinrichtung (16) im Kondensationsbetrieb der Kraftwerksanlage (1) zeitweise geöffnet ist.

13. Vorrichtung nach einem der Ansprüche 9 bis 12, wobei die Messeinrichtung (16) im Normalbetrieb der Kraftwerksanlage (1) entfernt ist.

14. Messeinrichtung (16) zur Messung des Reinheitsgrads eines Mediums und zur Verwendung in einer Vorrichtung zum Reinigen von Anlagenteilen (2.1.3, 2.2.3, 2.3, 2.4.2, 4.1a, 4.2, 4.3a, 4.3b, 4.4, 4.5a bis 4.5c) einer Kraftwerksanlage (1), umfassend mindestens eine Flanschverbindung (36a) zum Anschließen an einen Stutzen (34) mindestens eines durchströmbaren, betrieblichen Anlagenteils (4.1a, 4.2, 4.3a, 4.3b, 4.4, 4.5a bis 4.5c) und eine als Schleusenarmatur ausgebildete Doppelabsperrung (22), wobei in das durchströmbare, betriebliche Anlagenteil (4.1a, 4.2, 4.3a, 4.3b, 4.4, 4.5a bis 4.5c) eine Messvorrichtung (M) einführbar ist.

15. Messeinrichtung nach Anspruch 14, wobei die Doppelabsperrung (22) eingangs- und/oder ausgangsseitig mit mindestens einem elastischen Dichtelement (40) versehen ist.

16. Messeinrichtung nach Anspruch 15, wobei das Dichtelement (40) als Kammprofil-Dichtung ausgebildet ist.

17. Messeinrichtung nach einem der Ansprüche 14 bis 16, wobei die Doppelabsperrung (22) mit einem Absperrventil (32b) versehen ist.

18. Messeinrichtung nach einem der Ansprüche 14 bis 17, wobei die Doppelabsperrung (22) ein Auflager (44) umfasst.

19. Messeinrichtung nach Anspruch 18, wobei das Auflager (44) als eine Hülse mit einem umlaufenden Rand oder einem Konus ausgebildet ist.

20. Messeinrichtung nach Anspruch 18 oder 19, wobei das Auflager (44) zumindest teilweise einen angefasten Innenabsatz (44.1), insbesondere mit einem Winkel von größer 18°, aufweist.

21. Messeinrichtung nach einem der Ansprüche 18 bis 20, wobei das Auflager (44) zur Aufnahme einer Befestigung (46) der in das betriebliche Anlagenteil (4.1a, 4.2, 4.3a, 4.3b, 4.4, 4.5a bis 4.5c) einführbaren Messvorrichtung (M) dient.

22. Messeinrichtung nach Anspruch 21, wobei die Befestigung (46) der Messvorrichtung (M) im eingeschleusten Zustand gekapselt ist.

23. Messeinrichtung nach einem der Ansprüche 14 bis 22, wobei zum Ein- und Ausführen der Messvorrichtung (M) eine Gewindespindel (26) mit einem mechanischen Antrieb (30) vorgesehen ist.

24. Messeinrichtung nach einem der Ansprüche 14 bis 23, mit einer Halterung (20) zur Befestigung am betrieblichen Anlagenteil (4.1a, 4.2, 4.3a, 4.3b, 4.4, 4.5a bis 4.5c), wobei die Halterung (20) insbesondere als eine Sattelhalterung ausgebildet ist.
